# EUROPÄISCHE PATENTSCHRIFT

(11) **EP 1 019 190 B1**
(45) Veröffentlichungstag und Bekanntmachung des Hinweises auf die Patenterteilung: **01.12.2004**
(21) Anmeldenummer: 98948941.4
(22) Anmeldetag: 09.09.1998
(51) Int. Cl.: B01J 31/18, C07F 9/6571, C07F 15/04

(54) **KATALYSATOR, UMFASSEND WENIGSTENS EINEN NICKEL(O)KOMPLEX AUF BASIS EINES PHOSPHONITLIGANDEN UND VERFAHREN ZUR HERSTELLUNG VON NITRILEN**
CATALYST COMPRISING AT LEAST ONE PHOSPHONITE LIGAND BASED NICKEL (O) COMPLEX AND METHOD FOR THE PRODUCTION OF NITRILES
CATALYSEUR COMPRENANT AU MOINS UN COMPLEXE NICKEL(O) A BASE DE LIGANDS DE PHOSPHONITE ET PROCEDE DE PREPARATION DE NITRILES

(30) Priorität: 12.09.1997 DE 19740180
(43) Veröffentlichungstag der Anmeldung: 19.07.2000
(73) Patentinhaber: BASF AKTIENGESELLSCHAFT, 67056 Ludwigshafen (DE)
(72) Erfinder: FISCHER, Jakob, D-85414 Kirchdorf (DE); SIEGEL, Wolfgang, D-67117 Limburgerhof (DE)
(74) Vertreter: Kinzebach, Werner, Dr.
(86) Internationale Anmeldenummer: PCT/EP1998/005733
(87) Internationale Veröffentlichungsnummer: WO 1999/013983

(56) Entgegenhaltungen:
- WO-A-95/11977
- WO-A-95/29153
- WO-A-96/22968
- DE-A- 2 161 750
- DE-A- 4 321 194
- DE-A- 19 523 335

## Beschreibung

Die vorliegende Erfindung betrifft einen Katalysator, der einen Nickel(0)Komplex umfasst, welcher mindestens einen ein-, zweioder mehrzähnigen Phosphonitliganden umfasst, worin der Phosphor und eines der Sauerstoffatome der Phosphonitgruppe Teil eines 5-bis 8-gliedrigen Heterocyclus sind, sowie ein Verfahren zur Herstellung von Gemischen monoolefinischer C₅-Mononitrile durch katalytische Hydrocyanierung in Gegenwart eines solchen Katalysators.

Zur großtechnischen Herstellung von Polyamiden besteht weltweit ein großer Bedarf an α,ω-Alkylendiaminen, welche dabei als ein wichtiges Ausgangsprodukt dienen. α,ω-Alkylendiamine, wie z. B. das Hexamethylendiamin, werden fast ausschließlich durch Hydrierung der entsprechenden Dinitrile gewonnen. Fast alle großtechnischen Wege zur Herstellung von Hexamethylendiamin sind daher im Wesentlichen Varianten der Herstellung des Adipodinitrils, von dem jährlich weltweit etwa 1,0 Mio. Tonnen produziert werden.

In K. Weissermel, H.-J. Arpe, Industrielle Organische Chemie, 4. Auflage, VCH Weinheim, S. 266 ff. sind vier prinzipiell unterschiedliche Routen zur Herstellung von Adipinsäuredinitril beschrieben:
1. die dehydratisierende Aminierung der Adipinsäure mit Ammoniak in der Flüssig- oder Gasphase über intermediär gebildetes Diamid;
2. die indirekte Hydrocyanierung des 1,3-Butadiens über die zwischenstufe der 1,4-Dichlorbutene;
3. die Hydrodimerisierung von Acrylnitril in einem elektrochemischen Prozess; und
4. die direkte Hydrocyanierung von 1,3-Butadien mit Cyanwasserstoff.

Nach dem letztgenannten Verfahren erhält man in einer ersten Stufe durch Monoaddition ein Gemisch isomerer Pentennitrile, das in einer zweiten Stufe zu vorwiegend 3- und 4-Pentennitril isomerisiert wird. Anschließend wird in einer dritten Stufe durch anti-Markownikow-Cyanwasserstoffaddition an 4-Pentennitril das Adipinsäuredinitril gebildet. Die Umsetzung erfolgt dabei in der Flüssigphase in einem Lösungsmittel, wie z. B. Tetrahydrofuran, bei einer Temperatur im Bereich von 30 - 150 °C und drucklos. Dabei werden als Katalysatoren Nickelkomplexe mit Phosphin- bzw. Phosphit-Liganden und Metallsalz-Promotoren verwendet. In einen Heterocyclus integrierte Phosphonitliganden zur Stabilisierung des Nickels werden in dem oben genannten Review nicht beschrieben.

In "Applied Homogeneous Catalysis with Organometalic Compounds", Bd. 1, VCH Weinheim, S. 465 ff. wird allgemein die heterogen und homogen katalysierte Addition von Cyanwasserstoff an Olefine beschrieben. Dabei werden vor allem Katalysatoren auf Basis von Phosphin-, Phosphit- und Phosphinit-Komplexen des Nickels und Palladiums verwendet. Zur Herstellung von Adipinsäuredinitril durch Hydrocyanierung von Butadien werden vorwiegend Nikkel(0)-Phosphitkatalysatoren, ggf. in Gegenwart einer Lewis-Säure als Promotor verwendet. Allgemein lässt sich die Reaktion in die drei Schritte gliedern: 1. Synthese von Mononitrilen durch Hydrocyanierung von 1,3-Butadien; 2. Isomerisierung; 3. Synthese von Dinitrilen. Bei der Bildung des Monoadditionsproduktes erhält man ein Isomerengemisch, welches u. a. 3-Pentennitril und 2-Methyl-3-butennitril umfasst.

Übliche Katalysatoren für die Hydrocyanierung von 1,3-Butadien sind vor allem die bereits genannten Nickel(0)-Phosphitkatalysatoren.

C. A. Tolman et al. beschreiben in Organometallics 1984, 3, S. 33 f. die katalytische Hydrocyanierung von olefinen in Gegenwart von Nickel(0)-Phosphitkomplexen unter spezieller Berücksichtigung der Effekte von Lewis-Säuren auf die Cyanwasserstoffaddition.

In Advances in Catalysis, Band 33, 1985, Academic Press Inc., S. 1 f. wird übersichtsartig die homogen Nickel-katalysierte Hydrocyanierung von Olefinen beschrieben. Als Katalysatoren werden Nickel(0)-Komplexe mit Phosphin- und Phosphitliganden eingesetzt.

In J. Chem. Soc., Chem. Commun., 1991, S. 1292, werden chirale Aryldiphosphite als Liganden für Hydrocyanierungskatalysatoren beschrieben. Bei diesen Liganden ist die Phosphitgruppe über zwei ihrer Sauerstoffatome an die 3- und 3'-Positionen einer 2,2'-Binaphthyleinheit gebunden, mit der sie so einen 7-gliedrigen Heterocyclus bildet. Zusätzlich können zwei dieser Heterocyclen ebenfalls über eine 2,2'-Binaphthyleinheit zu einem zweizähnigen Chelatliganden verknüpft sein.

In J. Chem. Soc., Chem. Commun., 1991, S. 803 f., werden dazu analoge Chelatdiphosphit-Komplexe von Nickel(0) und Platin(0) beschrieben, wobei anstelle einer 2,2'-Binaphthyleinheit eine Biphenyleinheit eingesetzt wird.

Die WO 95/11077 beschreibt ein Verfahren zur Herstellung von Nickel(0)-Komplexen auf Basis von Phosphoratom-haltigen Liganden unter Einsatz von Ultraschall.

Die WO 95/28228 beschreibt ein Verfahren zur Hydrocyanierung von aliphatischen Monoolefinen, die gegebenenfalls zusätzlich eine nichtkonjugierte Nitrilgruppe oder eine nichtkonjugierte oder konjugierte Estergruppe aufweisen. Die eingesetzten Nickel(0)-Katalysatoren umfassen dabei ebenfalls zweizähnige Phosphitliganden, bei denen die Phosphitgruppen Teile von Aryl-anellierten Heterocyclen sind.

Die WO 95/29153 beschreibt ein Verfahren zur Hydrocyanierung von Monoolefinen, wobei Katalysatoren auf Basis von nullwertigem Nickel und einzähnigen Phosphitliganden eingesetzt werden. Bei diesen Liganden ist die Phosphitgruppe wiederum zusammen mit zwei ihrer Sauerstoffatome Teil eines Aryl-annellierten 7-gliedrigen Heterocyclus. Das dritte Sauerstoffatom der Phosphitgruppe trägt einen t.-Butyl-substituierten Phenylrest, welcher gegebenenfalls noch weitere Substituenten aufweisen kann.

Die WO 96/11182 beschreibt ein Verfahren zur Hydrocyanierung von aliphatischen, monoethylenisch ungesättigten Verbindungen, bei denen die ethylenische Doppelbindung nicht in Konjugation zu einer anderen ungesättigten Gruppe steht oder bei denen die ethylenische Doppelbindung in Konjugation zu einer Estergruppe steht. Dabei wird ein Nickel(0)-Katalysator auf Basis eines mehrzähnigen Phosphitliganden in Gegenwart einer Lewis-Säure als Promotor eingesetzt. Die Phosphitgruppen dieser mehrzähnigen Liganden sind dabei wiederum Bestandteile von Aryl-annellierten Heterocyclen und ggf. über Aryl-anellierte Gruppen verbrückt.

Die WO 96/22968 beschreibt ein Verfahren zur Hydrocyanierung diolefinischer Verbindungen und zur Isomerisierung der resultierenden, nichtkonjugierten 2-Alkyl-3-monoalkennitrile durch Umsetzung eines acyclischen, aliphatischen Diolefins mit einer Cyanwasserstoffquelle. Die Umsetzung findet dabei in der flüssigen Phase statt. Es werden Hydrocyanierungskatalysatoren eingesetzt, die denen in der WO 96/11182 beschriebenen analog sind.

Die US-A 5,512,695 hat einen der WO 95/28228 entsprechenden Offenbarungsgehalt.

Neben den zuvor beschriebenen Hydrocyanierungskatalysatoren auf Basis von ein-, zwei- und mehrzähnigen Phosphitliganden sind auch Katalysatoren auf Basis von Phosphinitliganden bekannt. In J. Am. Chem. Soc., 1994, 116, S. 9869 f. und in der US-A-5,484,902 sind Katalysatoren für die enantioselektive Hydrocyanierung aromatischer Vinylverbindungen auf der Basis von einem chiralen, nichtracemischen, zweizähnigen Chelatphosphinitliganden beschrieben. Als Ligand wird dabei vorzugsweise ein Phenyl-2,3-bis-O-(3,5-bis-(trifluormethyl)phenyl)phosphino-4,6-O-benzyliden-β-D-glucopyranosid eingesetzt.

Die US-A 5,523,453 beschreibt ein Verfahren zur Hydrocyanierung von Monoolefinen, welche gegebenenfalls zusätzlich eine Cyanogruppe aufweisen können, in Gegenwart einer Lewis-Säure als Promotor und einem Nickel(0)-Katalysator. Diese Katalysatoren weisen Liganden auf Basis von Chelatphosphiniten auf, bei denen zwei arylsubstituierte Phosphinitgruppen über ihr Sauerstoffatom und eine Aryl-anellierte Alkylenbrücke miteinander verknüpft sind.

Keine der zuvor genannten Literaturstellen beschreibt Hydrocyanierungskatalysatoren auf Basis von Phosphonitliganden, wobei die Phosphonitgruppe Teil eines 5- bis 8-gliedrigen Heterocyclus ist.

Die US-A 3,766,237 beschreibt ein Verfahren zur Bydrocyanierung ethylenisch ungesättigter Verbindungen, welche weitere funktionelle Gruppen, wie z. B. Nitrile, aufweisen können, in Gegenwart eines Nickelkatalysators. Diese Nickelkatalysatoren tragen vier Liganden der allgemeinen Formel M(X,Y,Z), wobei X, Y und Z unabhängig voneinander für einen Rest R oder OR stehen und R ausgewählt ist unter Alkyl- und Arylgruppen mit bis zu 18 Kohlenstoffatomen. Dabei werden jedoch nur Phosphine und Phosphite explizit genannt und in den Beispielen für die Hydrocyanierung eingesetzt. Dagegen ist nicht offenbart, dass Phosphonite, bei denen die Phosphonitgruppe Teil eines Heterocyclus ist, als Liganden für Nickel(0)-Hydrocyanierungskatalysatoren eingesetzt werden können.

Der vorliegenden Erfindung liegt die Aufgabe zugrunde, neue Katalysatoren auf Basis von nullwertigem Nickel zur Verfügung zu stellen, die bei der Hydrocyanierung von 1,3-Butadien-haltigen Kohlenwasserstoffgemischen sowie bei der Erst- und Zweitaddition von Cyanwasserstoff zur Herstellung von Adipodintril eine gute Selektivität und eine gute katalytische Aktivität aufweisen.

Überraschenderweise wurden nun Katalysatoren auf Basis von Nickel(0)-Komplexen gefunden, welche mindestens einen einzähnigen Phosphonitliganden umfassen, wobei die Phosphonitgrupp Teil eines 5- bis 8-gliedrigen Heterocyclus ist.

Gegenstand der vorliegenden Erfindung ist somit ein Katalysator, umfassend einen Nickel(O)komplex, mit mindestens einem einzähnigen Phosphonitliganden der allgemeinen Formel I 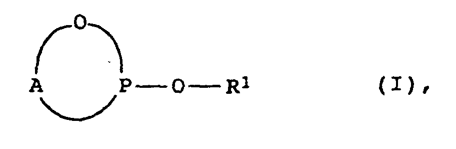 worin
- A: zusammen mit dem Teil der Phosphonitgruppe, an den es gebunden ist, für einen 5- bis 8-gliedrigen Heterocyclus steht, der gegebenenfalls zusätzlich ein-, zwei- oder dreifach mit Cycloalkyl, Aryl und/oder Hetaryl anelliert sein kann, wobei die anellierten Gruppen je einen, zwei oder drei Substituenten, ausgewählt unter Alkyl, Alkoxy, Halogen, Nitro, Cyano oder Carboxyl tragen können,
- R¹: für Alkyl, Aryl oder Hetaryl steht, welche einen, zwei oder drei der folgenden Substituenten: Alkyl, Cycloalkyl, Aryl, Alkoxy, Cycloalkyloxy, Acyl, Aryloxy, Halogen, Trifluormethyl, Nitro, Cyano, Carboxyl oder NE¹E² tragen können, wobei E¹ und E² gleich oder verschieden sein können und für Alkyl, Cycloalkyl oder Aryl stehen,
oder Salze und Mischungen davon.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₈-Alkyl-, bevorzugter C₁-C₆-Alkyl- und besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl.

Bei der Cycloalkylgruppe handelt es sich vorzugsweise um eine C₅-C₇-Cycloalkylgruppe, wie Cyclopentyl, Cyclohexyl oder Cycloheptyl.

Wenn die Cycloalkylgruppe substituiert ist, weist sie vorzugsweise 1, 2, 3, 4 oder 5, insbesondere 1, 2 oder 3 Substituenten, ausgewählt unter Alkyl, Alkoxy oder Halogen auf.

Aryl steht vorzugsweise für Phenyl, Tolyl, Xylyl, Mesityl, Naphthyl, Anthracenyl, Phenanthrenyl, Naphthacenyl und insbesondere für Phenyl oder Naphthyl.

Substituierte Arylreste weisen vorzugsweise 1, 2, 3, 4 oder 5, insbesondere 1, 2 oder 3 Substituenten, ausgewählt unter Alkyl, Alkoxy oder Halogen auf.

Hetaryl steht vorzugsweise für Pyridyl, Chinolinyl, Acridinyl, Pyridazinyl, Pyrimidinyl oder Pyrazinyl.

Substituierte Hetarylreste weisen vorzugsweise 1, 2 oder 3 Substituenten, ausgewählt unter Alkyl, Alkoxy oder Halogen auf.

Die obigen Ausführungen zu Alkyl-, Cycloalkyl- und Arylresten gelten entsprechend für Alkoxy-, Cycloalkyloxy- und Aryloxyreste.

Die Reste NE¹E² stehen vorzugsweise für N,N-Dimethyl, N,N-Diethyl, N,N-Dipropyl, N,N-Diisopropyl, N,N-Di-n-butyl, N,N-Di-t.-butyl, N,N-Dicyclohexyl oder N,N-Diphenyl.

Halogen steht für Fluor, Chlor, Brom und Iod, bevorzugt für Fluor, Chlor und Brom.

Eine bevorzugte Ausführungsform der Erfindung sind Katalysatoren, die mindestens einen Phosphonitliganden der Formel I umfassen, wobei A zusammen mit dem Teil der Phosphonitgruppe, an den es gebunden ist, für einen 5- oder 6-gliedrigen Heterocyclus steht, der gegebenenfalls ein- oder zweifach mit Aryl und/oder Hetaryl anelliert sein kann, wobei die anellierten Gruppen einen, zwei oder drei der zuvor angegebenen Substituenten tragen können.

Der Rest A steht dann z. B. für einen 2,2'-Biphenylen-, 2,2'-Binaphthylen- oder 2,3-Xylylen-Rest, der 1, 2 oder 3 Substituenten, ausgewählt unter Alkyl, Alkoxy oder Halogen, tragen kann. Alkyl steht dabei vorzugsweise für C₁-C₄-Alkyl und insbesondere für t.-Butyl. Alkoxy steht dabei vorzugsweise für C₁-C₄-Alkoxy und insbesondere für Methoxy. Halogen steht insbesondere für Fluor, Chlor oder Brom.

Insbesondere steht A für einen Rest der Formeln IV.1, IV.2 oder IV.3: 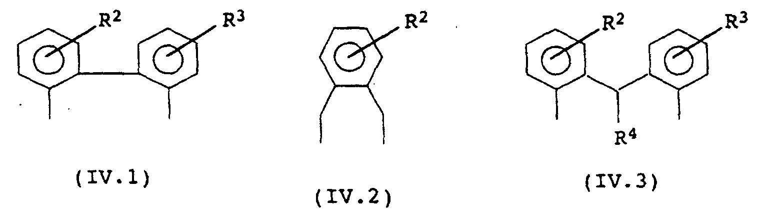 worin
- R² und R³: unabhängig voneinander für Wasserstoff, Alkyl, Alkoxy, Halogen, Trifluormethyl, Nitro oder Cyano stehen, und
- R⁴: für Wasserstoff, Alkyl, vorzugsweise Methyl, oder Aryl, vorzugsweise Phenyl, steht, welches gegebenenfalls mit Alkyl, Alkoxy, Halogen, Trifluormethyl, Nitro oder Cyano substituiert sein kann.

Nach einer weiteren bevorzugten Ausführungsform umfassen die erfindungsgemäßen Katalysatoren mindestens einen Phosphitliganden der Formel I, wobei
- R¹: für Phenyl oder Naphthyl steht, welches einen, zwei oder drei der folgenden Substituenten: Alkyl, Alkoxy, Halogen, Nitro, Cyano, Carboxyl oder NE¹E² tragen kann, wobei E¹ und E² die zuvor angegebenen Bedeutungen besitzen.

Wenn R¹ für Phenyl oder Naphthyl steht, so weist dieses vorzugsweise 1, 2 oder 3 Substituenten, ausgewählt unter C₁-C₄-Alkyl, C₁-C₄-Alkoxy oder Halogen auf. Insbesondere sind die Substituenten ausgewählt unter t.-Butyl, Methoxy, Trifluormethyl, Fluor, Chlor und Brom.

Nach einer geeigneten Ausführungsform sind die Phosphonitliganden der Formel I ausgewählt unter Liganden der Formel Ia bis Ie 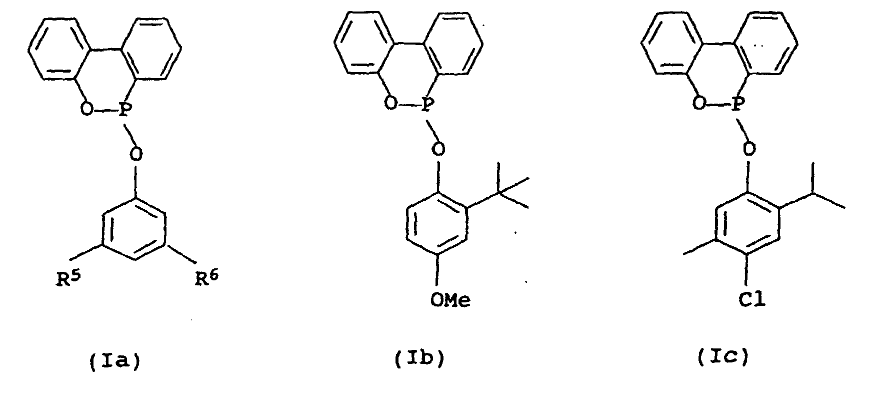 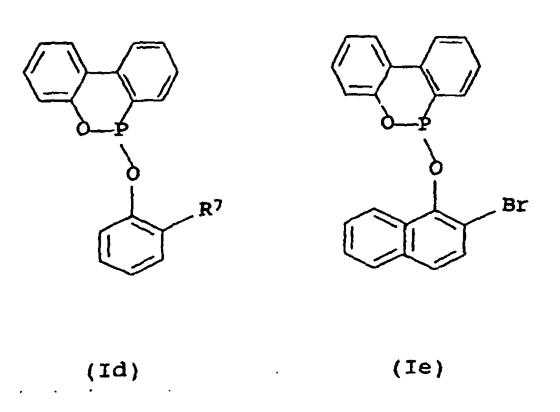 worin
- R⁵ und R⁶: unabhängig voneinander für Wasserstoff oder Trifluormethyl stehen,
- R⁷: für Fluor oder Trifluormethyl steht.

Die erfindungsgemäßen Katalysatoren können einen oder mehrere der Phosphitliganden der Formel I aufweisen. Zusätzlich zu den zuvor beschriebenen Liganden der allgemeinen Formel I können sie noch wenigstens einen weiteren Liganden, der ausgewählt ist unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃ sowie ein-, zwei- und mehrzähnigen Phosphin-, Phosphinit-, Phosphonit- und Phosphitliganden aufweisen. Diese weiteren Liganden können ebenfalls ein-, zwei- oder mehrzähnig sein und an das nullwertige Nickel koordinieren. Geeignete weitere phosphorhaltige Liganden sind z. B. die zuvor als Stand der Technik beschriebenen Phosphin-, Phosphinit-, und Phosphitliganden.

Zur Herstellung der erfindungsgemäß eingesetzten Phosphonitliganden der Formel I kann man z. B. eine Hydroxylgruppen-haltige Verbindung der Formel V mit einem Phosphortrihalogenid, bevorzugt PCl₃, zu einer Verbindung der Formel VI und diese dann mit einer Hydroxylgruppen-haltigen Verbindung der Formel HOR¹ gemäß folgendem Schema 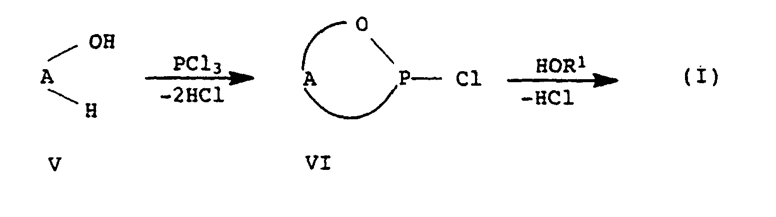 umsetzen,
wobei A und R¹ die zuvor angegebenen Bedeutungen besitzen. Ein solches Verfahren wird in Phosphorus and Sulfur, 1987, Bd. 31, S. 71 ff. für den Aufbau von 6H-Dibenz[c,e][1,2]oxaphosphorin-Ringsystemen beschrieben.

Geeignete Alkohole der Formel V sind z. B. Biphenyl-2-ol, Binaphthyl-2-ol etc.

Geeignete Alkohole der Formel HOR¹ sind z. B. 2-tert-Butyl-4-methylphenol, 2-tert-Butylphenol, 4-tert-Butylphenol, 2,6-Di-tertbutyl-4-methylphenol, 2,4-Di-tert-butylphenol, 2,6-Di-tert-butylphenol, 2,4-Dimethylphenol, 2,5-Dimethylphenol, 2,6-Dimethylphenol, 3,4-Dimethylphenol, 3,5-Dimethylphenol, 2-Ethylphenol, 3-Ethylphenol, 4-Ethylphenol, 5-Isopropyl-2-methylphenol, m-Kresol, o-Kresol, p-Kresol, 1-Naphthol, 2-Naphthol, Phenol, 1-Brom-2-naphthol, 3-Bromphenol, 5-Chlorchinolin-8-ol, 4-Chlor-3,5-dimethylphenol, 2-Chlor-5-methylphenol, 4-Chlor-3-methylphenol, 2-Chlor-6-nitrophenol, 2-Clorphenol, 3-Chlorphenol, 4-Chlorphenol, 4-Chlorresorcin, 2,3-Dichlorphenol, 2,4-Dichlorphenol, 2,5-Dichlorhphenol, 2,6-Dichlorphenol, 3,4-Dichlorhphenol, 2-Fluorphenol, 3-Fluorphenol, 4-Fluorphenol, 3-Methyl-4-nitrophenol, 2-Nitroanisol, 4-Nitrobrenzcatechin, 2-Nitrophenol, 3-Nitrophenol, 2-Methoxymethylphenol, 2-Methoxy-4-methylphenol, 2-Methoxyphenol, 3-Methoxyphenol, 4-Methoxyphenol. Bevorzugte Alkohole der Formel HOR¹ sind 2,6-Di-tert-butyl-4-methylphenol, 2,4-Di-tert-butylphenol, 2,6-Di-tert-butylphenol, Phenol, 2-Fluorphenol, 3-Fluorphenol, 4-Fluorphenol, 4-Nitrobrenzcatechin, 2-Methoxy-4-methylphenol, 2-Trifluormethylphenol, 3,5-Bis(trifluormethyl)phenol, 4-Cyanophenol, etc..

Die Verbindungen der Formel VI können gewünschtenfalls isoliert und einer Reinigung, z. B. durch Destillation, unterworfen werden. Die Umsetzung der Verbindung der Formel V zu einer Verbindung der Formel VI verläuft im Allgemeinen bei einer erhöhten Temperatur in einem Bereich von etwa 40 bis etwa 200 °C, wobei die Umsetzung auch unter sukzessiver Temperaturerhöhung geführt werden kann. Zusätzlich kann zu Beginn der Reaktion oder nach einer gewissen Reaktionsdauer eine Lewis-Säure, wie z. B. Zinkchlorid oder Aluminiumchlorid, als Katalysator zugesetzt werden. Die weitere Umsetzung der Verbindungen der Formel VI zu den erfindungsgemäß eingesetzten Phosphonitliganden der Formel I erfolgt im Allgemeinen in Gegenwart einer Base, z. B. einem aliphatischen Amin, wie Diethylamin, Dipropylamin, Dibutylamin, Trimethylamin, Tripropylamin und vorzugsweise Triethylamin oder Pyridin.

Vorteilhafterweise gelingt die Herstellung der erfindungsgemäß eingesetzten Phosphonitliganden der Formel I ohne Verwendung von Magnesium- oder Lithium-organischen Verbindungen. Die einfache Reaktionssequenz erlaubt eine breite Variationsmöglichkeit der Liganden. Die Darstellung gelingt somit effizient und ökonomisch aus leicht zugängigen Edukten.

Zur Herstellung der erfindungsgemäßen Katalysatoren kann man mindestens einen Phosphonitliganden der Formel I mit Nickel oder einer Nickelverbindung in Gegenwart eines Reduktionsmittels oder einem Nickelkomplex in einem inerten Lösungsmittel zur Reaktion bringen. Geeignete Nickelverbindungen sind dabei z. B. Verbindungen, in denen das Übergangsmetall eine Oxidationsstufe höher als 0 einnimmt, und die bei der Umsetzung mit dem Phosphonitliganden der Formel I, gegebenenfalls in Gegenwart eines geeigneten Reduktionsmittels, in situ reduziert werden. Dazu zählen z. B. die Halogenide, bevorzugt die Chloride, und die Acetate der zuvor genannten Übergangsmetalle. Dabei wird bevorzugt NiCl₂ eingesetzt. Geeignete Reduktionsmittel sind z. B. Metalle, bevorzugt Alkalimetalle, wie Na und K, Aluminium, Zink sowie Trialkylaluminiumverbindungen.

Werden zur Herstellung der Phosponit-Nickel(0)-Komplexe bereits Komplexverbindungen des Übergangsmetalls eingesetzt, so liegt in diesen das Übergangsmetall vorzugsweise bereits nullwertig vor. Bevorzugt werden zur Herstellung Komplexe mit Liganden eingesetzt, die den zuvor genannten, zusätzlichen Liganden der erfindungsgemäßen Komplexe entsprechen. In diesem Falle erfolgt die Herstellung durch teilweisen oder vollständigen Ligandenaustausch mit den zuvor beschriebenen Phosphonitliganden der Formel I.

Nach einer geeigneten Ausführungsform des erfindungsgemäßen Verfahrens ist der Nickelkomplex Bis(1,5-cyclooctadien)nickel(0).

Geeignete inerte Lösungsmittel zur Herstellung der Nickel(0)-Komplexe sind beispielsweise Aromaten, wie Benzol, Toluol, Ethylbenzol, Chlorbenzol, Ether, vorzugsweise Diethylether und Tetrahydrofuran, oder Halogenalkane, beispielsweise Dichlormethan, Chloroform, Dichlorethan und Trichlorethan. Die Temperatur liegt dabei in einem Bereich von -70 °C bis 150 °C, vorzugsweise von 0 °C bis 100 °C, besonders bevorzugt etwa bei Raumtemperatur.

Wird zur Herstellung der Phosphonit-Nickel(0)-Komplexe elementares Nickel eingesetzt, so liegt dieses vorzugsweise als Pulver vor. Die Umsetzung von Nickel und Phosphonitligand erfolgt vorzugsweise in einem Produkt der Hydrocyanierungsreaktion als Lösungsmittel, z. B. in einem Gemisch monoolefinischer C₅-Mononitrile oder bevorzugt in 3-Pentennitril. Gegebenenfalls kann auch der Ligand als Lösungsmittel eingesetzt werden. Die Temperatur liegt in einem Bereich von etwa 0 bis 150 °C, bevorzugt 60 bis 100 °C.

Ein weiterer Gegenstand der Erfindung ist ein Verfahren zur Herstellung von Gemischen monoolefinischer C₅-Mononitrile mit nichtkonjugierter C=C- und C≡N-Bindung durch katalytische Hydrocyanierung eines 1,3-Butadien-haltigen Kohlenwasserstoffgemisches, das dadurch gekennzeichnet ist, dass die Hydrocyanierung in Gegenwart mindestens eines der zuvor beschriebenen erfindungsgemäßen Katalysatoren erfolgt.

Vorzugsweise wird zur Herstellung von monoolefinischen C₅-Mononitrilen nach dem erfindungsgemäßen Verfahren ein Kohlenwasserstoffgemisch eingesetzt, das einen 1,3-Butadien-Gehalt von mindestens 10 Vol.-%, bevorzugt mindestens 25 Vol.-%, insbesondere mindestens 40 Vol.-%, aufweist.

Zur Herstellung von Gemischen monoolefinischer C₅-Mononitrile, die z.B. 3-Pentennitril und 2-Methyl-3-butennitril enthalten und die als Zwischenprodukte für die Weiterverarbeitung zur Adipodinitril geeignet sind, kann man reines Butadien oder 1,3-Butadien-haltige Kohlenwasserstoffgemische einsetzen.

1,3-Butadien-haltige Kohlenwasserstoffgemische sind in großtechnischem Maßstab erhältlich. So fällt z.B. bei der Aufarbeitung von Erdöl durch Steamcracken von Naphtha ein als C₄-Schnitt bezeichnetes Kohlenwasserstoffgemisch mit einem hohen Gesamtolefinanteil an, wobei etwa 40 % auf 1,3-Butadien und der Rest auf Monoolefine und mehrfach ungesättigte Kohlenwasserstoffe sowie Alkane entfällt. Diese Ströme enthalten immer auch geringe Anteile von im Allgemeinen bis zu 5 % an Alkinen, 1,2-Dienen und Vinylacetylen.

Reines 1,3-Butadien kann z. B. durch extraktive Destillation aus technisch erhältlichen Kohlenwasserstoffgemischen isoliert werden.

C₄-Schnitte werden gegebenenfalls von Alkinen, wie z. B. Propin oder Butin, von 1,2-Dienen, wie z. B. Propadien, und von Alkeninen, wie z. B. Vinylacetylen, im Wesentlichen befreit. Ansonsten werden u.U. Produkte erhalten, bei denen eine C=C-Doppelbindung in Konjugation mit der C≡N-Bindung steht. Aus "Applied Homogeneous Catalysis with Organometalic Compounds", Bd. 1, VCH Weinheim, S. 479 ist bekannt, dass das bei der Isomerisierung von 2-Methyl-3-butennitril und 3-Pentennitril entstehende, konjugierte 2-Pentennitril als ein Reaktionsinhibitor für die Zweitaddition von Cyanwasserstoff zu Adipinsäuredinitril wirkt. Es wurde festgestellt, dass die oben genannten, bei der Hydrocyanierung eines nicht vorbehandelten C₄-Schnittes erhaltenen konjugierten Nitrile auch als Katalysatorgifte für den ersten Reaktionsschritt der Adipinsäureherstellung, die Monoaddition von Cyanwasserstoff, wirken.

Daher entfernt man gegebenenfalls aus dem Kohlenwasserstoffgemisch solche Komponenten teilweise oder vollständig, die bei katalytischer Hydrocyanierung Katalysatorgifte ergeben, insbesondere Alkine, 1,2-Diene und Gemische davon. Zur Entfernung dieser Komponenten wird der C₄-Schnitt vor der Addition von Cyanwasserstoff einer katalytischen Teilhydrierung unterzogen. Diese Teilhydrierung erfolgt in Gegenwart eines Hydrierungskatalysators, der befähigt ist, Alkine und 1,2-Diene selektiv neben anderen Dienen und Monoolefinen zu hydrieren.

Geeignete heterogene Katalysatorsysteme umfassen im Allgemeinen eine Übergangsmetallverbindung auf einem inerten Träger. Geeignete anorganische Träger sind die hierfür üblichen Oxide, insbesondere Silicium- und Aluminiumoxide, Alumosilikate, Zeolithe, Carbide, Nitride etc. und deren Mischungen. Bevorzugt werden als Träger Al₂O₃, SiO₂ und deren Mischungen verwendet. Insbesondere handelt es sich bei den verwendeten heterogenen Katalysatoren um die in den US-A-4,587,369; US-A-4,704,492 und US-A-4,493,906 beschriebenen, auf die hier in vollem Umfang Bezug genommen wird. Weiterhin geeignete Katalysatorsysteme auf Cu-Basis werden von der Fa. Dow Chemical als KLP-Katalysator vertrieben.

Die Addition von Cyanwasserstoff an 1,3-Butadien oder ein 1,3-Butadien-haltiges Kohlenwasserstoffgemisch, z. B. einen vorbehandelten, teilhydrierten C₄-Schnitt, kann kontinuierlich, semikontinuierlich oder diskontinuierlich erfolgen.

Nach einer geeigneten Variante des erfindungsgemäßen Verfahrens erfolgt die Addition des Cyanwasserstoffs kontinuierlich. Geeignete Reaktoren für die kontinuierliche Umsetzung sind dem Fachmann bekannt und werden z. B. in Ullmanns Enzyklopädie der technischen Chemie, Bd. 1, 3. Aufl., 1951, S. 743 ff. beschrieben. Vorzugsweise wird für die kontinuierliche Variante des erfindungsgemäßen Verfahrens eine Rührkesselkaskade oder ein Rohrreaktor verwendet.

Gemäß einer bevorzugten Variante des erfindungsgemäßen Verfahrens erfolgt die Addition des Cyanwasserstoffs an 1,3-Butadien oder ein 1,3-Butadien-haltiges Kohlenwasserstoffgemisch semikontinuierlich.

Das semikontinuierliche Verfahren umfasst:
a) Befüllen eines Reaktors mit dem Kohlenwasserstoffgemisch, gegebenenfalls einem Teil des Cyanwasserstoffs und einem gegebenenfalls in situ erzeugten, erfindungsgemäßen Hydrocyanierungskatalysator sowie gegebenenfalls einem Lösungsmittel,
b) Umsetzung des Gemisches bei erhöhter Temperatur und erhöhtem Druck, wobei bei semikontinuierlicher Fahrweise Cyanwasserstoff nach Maßgabe seines Verbrauchs eingespeist wird,
c) Vervollständigung des Umsatzes durch Nachreagieren und anschließende Aufarbeitung.

Geeignete druckfeste Reaktoren sind dem Fachmann bekannt und werden z. B. in Ullmanns Enzyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann. Für die obigen Schritte gilt vorzugsweise folgendes zu beachten:

### Schritt a):

Der druckfeste Reaktor wird vor Beginn der Reaktion mit dem teilhydrierten C₄-Schnitt, Cyanwasserstoff einem Hydrocyanierungskatalysator sowie ggf. einem Lösungsmittel befüllt. Geeignete Lösungsmittel sind dabei die zuvor bei der Herstellung der erfindungsgemäßen Katalysatoren genannten, bevorzugt aromatischen Kohlenwasserstoffe, wie Toluol und Xylol, oder Tetrahydrofuran.

### Schritt b):

Die Umsetzung des Gemisches erfolgt im Allgemeinen bei erhöhter Temperatur und erhöhtem Druck. Dabei liegt die Reaktionstemperatur im Allgemeinen in einem Bereich von etwa 0 bis 200°C, bevorzugt etwa 50 bis 150 °C, insbesondere 70 bis 120 °C. Der Druck liegt im Allgemeinen in einem Bereich von etwa 0,1 bis 200 bar, bevorzugt etwa 1 bis 200 bar, insbesondere bevorzugt etwa 1 bis 100 bar, speziell 1 bis 50 bar, spezieller bevorzugt 1 bis 15 bar. Dabei wird während der Reaktion Cyanwasserstoff nach Maßgabe seines Verbrauchs eingespeist, wobei der Druck im Autoklaven im Wesentlichen konstant bleibt. Die Reaktionszeit beträgt etwa 30 Minuten bis 5 Stunden.

### Schritt c):

Zur Vervollständigung des Umsatzes kann sich an die Reaktionszeit eine Nachreaktionszeit von 0 Minuten bis etwa 5 Stunden, bevorzugt etwa 1 Stunde bis 3,5 Stunden anschließen, in der kein Cyanwasserstoff mehr in den Autoklaven eingespeist wird. Die Temperatur wird in dieser Zeit im wesentlichen konstant auf der zuvor eingestellten Reaktionstemperatur belassen. Die Aufarbeitung erfolgt nach gängigen Verfahren und umfasst die Abtrennung des nicht umgesetzten 1,3-Butadiens und des nicht umgesetzten Cyanwasserstoffs, z. B. durch Waschen oder Extrahieren und die destillative Aufarbeitung des übrigen Reaktionsgemisches zur Abtrennung der Wertprodukte und Rückgewinnung des noch aktiven Katalysators.

Gemäß einer weiteren geeigneten Variante des erfindungsgemäßen Verfahrens erfolgt die Addition des Cyanwasserstoffs an das 1,3-Butadien-haltige Kohlenwasserstoffgemisch diskontinuierlich. Dabei werden im Wesentlichen die bei semikontinuierlichen Verfahren beschriebenen Reaktionsbedingungen eingehalten, wobei in Schritt b) kein zusätzlicher Cyanwasserstoff eingespeist, sondern dieser komplett vorgelegt wird.

Allgemein lässt sich die Herstellung von Adipinsäuredinitril aus einem Butadien-haltigen Gemisch durch Addition von 2 Moläquivalenten Cyanwasserstoff in drei Schritte gliedern:
1. Herstellung von C₅-Monoolefingemischen mit Nitrilfunktion.
2. Isomerisierung des in diesen Gemischen enthaltenen 2-Methyl-3-butennitrils zu 3-Pentennitril und Isomerisierung des so gebildeten und des in den Gemischen bereits aus Schritt 1 enthaltenen 3-Pentennitrils zu verschiedenen n-Pentennitrilen. Dabei soll ein möglichst hoher Anteil an 3-Pentennitril bzw. 4-Pentennitril und ein möglichst geringer Anteil an konjugiertem und gegebenenfalls als Katalysatorgift wirksamen 2-Pentennitril und 2-Methyl-2-butennitril gebildet werden.
3. Herstellung von Adipinsäuredinitril durch Addition von Cyanwasserstoff an das in Schritt 2 gebildete 3-Pentennitril welches zuvor "in situ" zu 4-Pentennitril isomerisiert wird. Als Nebenprodukte treten dabei z. B. 2-Methyl-glutarodinitril aus der Markownikow-Addition von Cyanwasserstoff an 4-Pentennitril oder der anti-Markownikow-Addition von Cyanwasserstoff an 3-Pentennitril und Ethylsuccinodinitril aus der Markownikow-Addition von Cyanwasserstoff an 3-Pentennitril auf.

Vorteilhafterweise eignen sich die erfindungsgemäßen Katalysatoren auf Basis von Phosphonitliganden auch für die Stellungs- und Doppelbindungsisomerisierung in Schritt 2 und/oder die Zweitaddition von Cyanwasserstoff in Schritt 3.

Nach einer geeigneten Ausführungsform des erfindungsgemäßen Verfahrens beträgt das bei der Monoaddition von Cyanwasserstoff an das 1,3-Butadien-haltige Kohlenwasserstoffgemisch erhaltene Mengenverhältnis von 3-Pentennitril zu 2-Methyl-3-butennitril mindestens 1,9:1, bevorzugt mindestens 2,1:1.

Vorteilhafterweise zeigen die erfindungsgemäß eingesetzten Katalysatoren nicht nur eine hohe Selektivität im Bezug auf die bei der Hydrocyanierung von 1,3-Butadien-haltigen Kohlenwasserstoffgemischen erhaltenen Monoadditionsprodukte, sondern sie können bei der Hydrocyanierung auch mit einem Überschuss an Cyanwasserstoff versetzt werden, ohne dass es zu einer merklichen Abscheidung von inaktiven Nickel(II)-Verbindungen, wie z. B. Nikkel(II)-Cyanid, kommt. Im Gegensatz zu bekannten Hydrocyanierungskatalysatoren auf Basis nicht-komplexer Phosphin- und Phosphitliganden eignen sich die Katalysatoren der Formel I somit nicht nur für kontinuierliche Hydrocyanierungsverfahren, bei denen ein Cyanwasserstoffüberschuss im Reaktionsgemisch im Allgemeinen wirkungsvoll vermieden werden kann, sondern auch für semikontinuierliche Verfahren und Batch-Verfahren, bei denen im Allgemeinen ein starker Cyanwasserstoffüberschuss vorliegt. Somit weisen die erfindungsgemäß eingesetzten Katalysatoren und die auf ihnen basierenden Verfahren zur Hydrocyanierung im Allgemeinen höhere Katalysatorrückführungsraten und längere Katalysatorstandzeiten auf als bekannte Verfahren. Dies ist neben einer besseren Wirtschaftlichkeit auch unter ökologischen Aspekten vorteilhaft, da das aus dem aktiven Katalysator mit Cyanwasserstoff gebildete Nickelcyanid stark giftig ist und unter hohen Kosten aufgearbeitet oder entsorgt werden muss.

Neben der Hydrocyanierung von 1,3-Butadien-haltigen Kohlenwasserstoffgemischen eignen sich die Katalysatoren der Formel I im Allgemeinen für alle gängigen Hydrocyanierungsverfahren. Dabei sei insbesondere die Hydrocyanierung von nichtaktivierten Olefinen, z. B. von Styrol und 3-Pentennitril, genannt.

Die zuvor beschriebenen Katalysatoren, die chirale Phosphonitliganden der Formel I umfassen, eignen sich zur enantioselektiven Hydrocyanierung.

Die Erfindung wird anhand der folgenden, nicht einschränkenden Beispiele näher erläutert.

### Beispiele

### A) Herstellung der Liganden Ia bis Ig

### Beispiel 1:

### Herstellung von Ligand Ia

206 g (1,5 mol) Phosphortrichlorid und 204 g (1,2 mol) Biphenyl-2-ol werden unter Rühren in einer Argonatmosphäre langsam auf 50 °C und innerhalb von 8 Stunden weiter auf 140 °C erhitzt. Bei starker Chlorwasserstoffentwicklung färbt sich die Lösung gelb. Nach Abkühlen auf 120 °C fügt man eine katalytische Menge an Zinkchlorid (1,2 g; 17 mmol) zu und erhitzt 24 Stunden bei 140 °C. Bei anschließender Destillation geht das Reaktionsprodukt 6-Chlor-(6H)-dibenz[c,e][1,2]-oxaphosphorin bei einem Siedepunkt von 132 °C (0,2 mbar) über. Ausbeute: 194,8 g (69 %) weiße Kristalle; ³¹P-NMR-Spektrum: δ (ppm) 134,5.

0,1 mol dieses Produkts werden in einer Argonatmosphäre zusammen mit 0,1 mol Phenol in 50 ml Toluol vorgelegt. Bei Raumtemperatur werden 0,1 mol Triethylamin (über KOH getrocknet) zugetropft. Anschließend rührt man eine Stunde bei 40 °C nach. Das entstandene Triethylammoniumhydrochlorid wird abfiltriert und die flüchtigen Bestandteile im Hochvakuum entfernt. Zurück bleibt ein durchsichtiges öliges Produkt (100 % Rohausbeute). Zur weiteren Reinigung kann der Ligand mit n-Hexan gewaschen oder umkristallisiert werden.

³¹P-NMR-Spektrum: δ (ppm) 127,4; Reinheit Rohprodukt: 97 % ¹H-NMR: entspricht dem für Ligand Ia erwarteten Spektrum

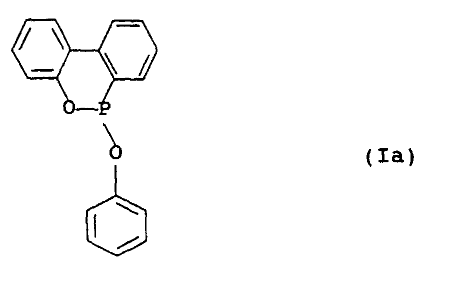

### Beispiel 2:

### Herstellung von Ligand Ib

Analog der im Beispiel 1 angegebenen Synthesevorschrift erfolgt die Herstellung des Liganden Ib. 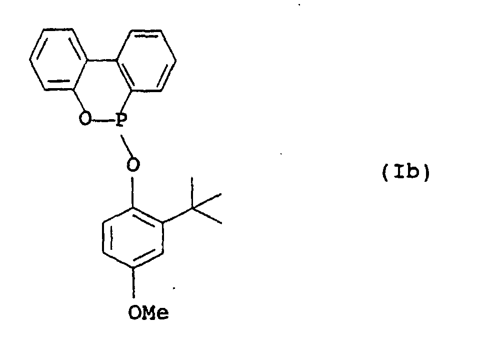 ³¹P-NMR-Spektrum: δ (ppm) 125,3
¹H-NMR entspricht dem Strukturvorschlag
Reinheit Rohprodukt: > 90 %

### Beispiel 3:

### Herstellung von Ligand Ic

Analog der in Beispiel 1 angegebenen Synthesevorschrift erfolgt die Herstellung des Liganden Ic. 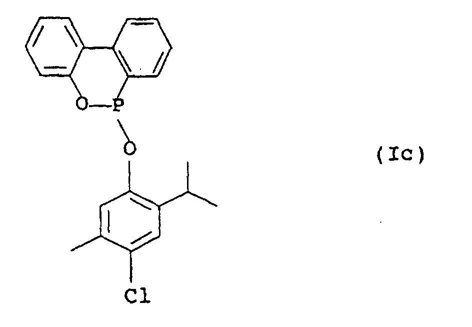 ³¹P-NMR-Spektrum: δ (ppm) 128,02
¹H-NMR-Spektrum: entspricht dem Strukturvorschlag
¹³C-NMR-Spektrum: entspricht dem Strukturvorschlag
Reinheit Rohprodukt: > 95 %

### Beispiel 4:

### Herstellung von Ligand Id

Analog der in Beispiel 1 angegebenen Synthesevorschrift erfolgt die Herstellung des Liganden Id. 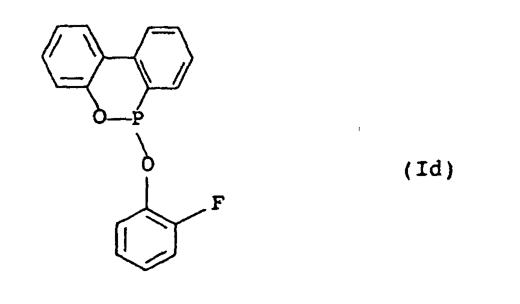 ³¹P-NMR-Spektrum: δ (ppm) 131,44
¹H-NMR-Spektrum: entspricht dem Strukturvorschlag
¹³C-NMR-Spektrum: entspricht dem Strukturvorschlag
Reinheit Rohprodukt: > 87 %

### Beispiel 5:

### Herstellung von Ligand Ie

Analog der in Beispiel 1 angegebenen Synthesevorschrift erfolgt die Herstellung des Liganden Ie. 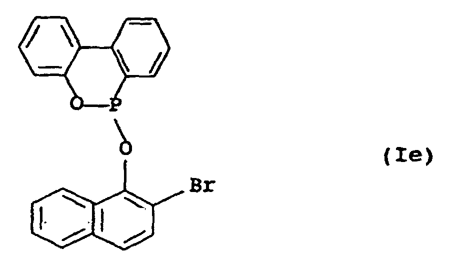 ³¹P-NMR-Spektrum: δ (ppm) 131,41
¹H-NMR-Spektrum: entspricht dem Strukturvorschlag
¹³C-NMR-Spektrum: entspricht dem Strukturvorschlag
Reinheit Rohprodukt: > 88 %

### Beispiel 6:

### Herstellung des Liganden If (nicht erfindungsgemäß)

Analog der in Beispiel 1 angegebenen Synthesevorschrift erfolgt die Herstellung von 6-Chlor-(6H)-dibenz[c,e][1,2]-oxaphosphorin.

40 g (0,177 mol) 6-Chlor-(6H)-dibenz[c,e][1,2]-oxaphosphorin werden unter Argon zusammen mit 31,7 g (0,088 mol) 5,5'-Dimethoxy-3,3'di-t.-butyl-2,2'-biphenol in 400 ml Toluol vorgelegt. Bei Raumtemperatur werden 20,24 g (0,2 mol) Triethylamin (über KOH getrocknet) zugetropft. Anschließend rührt man 120 Minuten bei 90 °C nach. Das entstandene Triethylammoniumhydrochlorid wird abfiltriert und der Filterrückstand zur Vervollständigung der Ausbeute mit Tetrahydrofuran nachgewaschen. Von den vereinigten organischen Phasen werden die flüchtigen Bestandteile in Hochvakuum entfernt. Als Produkt erhält man den Liganden If in 100 % Rohausbeute. Der weißgelbe Feststoff wird zunächst mit n-Hexan und dann mit Diethylether gewaschen. 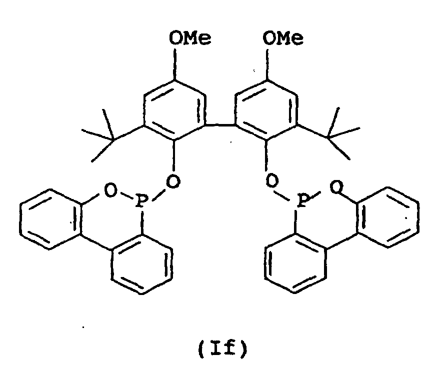 ³¹P-NMR-Spektrum: δ (ppm) 128,14

### Beispiel 7:

### Herstellung von Ligand Ig (nicht erfindungsgemäß)

Analog der in Beispiel 6 angegebenen Synthesevorschrift erfolgt die Herstellung des Liganden Ig. Das erhaltene Rohprodukt weist eine braune Farbe auf und ist leicht klebrig. Es wird zur Reinigung 12 Stunden in n-Hexan kräftig gerührt. Nach Abtrennen der überstehenden Hexanlösung erhält man den Liganden Ig als weißes Pulver. 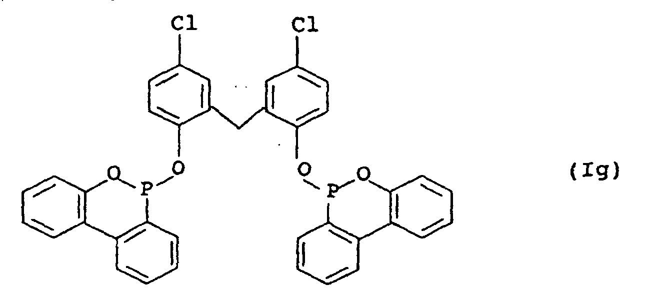 ³¹P-NMR-Spektrum: δ (ppm) 128,41
¹H-NMR-Spektrum: entspricht dem Strukturvorschlag
Reinheit Rohprodukt: > 89 %
Hydrocyanierungen und Isomerisierungen

### Beispiel 8 (erfindungsgemäß):

### Semikontinuierliche Hydrocyanierung von C₄-Schnitt unter Einsatz von Ligand Ia

**Tabelle 1:**

| Zusammensetzung des C₄-Schnittes | |
|---|---|
| Verbindung | Vol.-% |
| 1,3-Butadien | 40,50 |
| cis-2-Buten | 2,65 |
| trans-2-Buten | 4,30 |
| Isobuten | 30,20 |
| 1-Buten | 14,30 |
| Isobutan | 1,10 |
| n-Butan | 2,90 |
| Propin | 0,50 |
| Kohlendioxid | 0,10 |
| Vinylacetylen | 0,35 |

In einem Glasautoklaven werden unter Argon bei Raumtemperatur 0,41 g (1,5 mmol) Bis(1,5-cyclooctadien)nickel(0), 1,75 g (6 mmol) Ligand Ia und 6 g Toluol miteinander vermengt, wobei sich der Reaktionsansatz sofort gelb-braun färbt. Nach etwa einer Stunde wird eine Mischung aus 20 g C₄-Schnitt mit einer Zusammensetzung gemäß Tabelle 1 in 40 g Toluol zugegeben. Der Glasautoklave wird fest verschlossen und die Reaktionsmischung auf 80 °C erhitzt, wobei sich ein Anfangsdruck von 3,5 bar einstellt. Über einen Zeitraum von 120 Minuten wird eine Mischung aus 4,0 g (0,15 mol) frisch destillierter Blausäure in 40 g Toluol kontinuierlich zudosiert. Danach ist der Druck auf 2,9 bar gefallen. Anschließend lässt man zum Vervollständigen der Reaktion noch 240 Minuten bei etwa 80 °C nachreagieren. Zum Nachspülen des Reaktionsaustrages wird Toluol verwendet. Der Verlauf der Reaktion wird über Druck- und Temperaturmessung verfolgt.

Bei einer anschließenden Cyanid-Bestimmung nach Volhard wird ein Cyanwasserstoffumsatz von 86,7 % ermittelt.

GC-Analytik (Säule: 30 m Stabil-Wachs, Temperaturprogramm: 5 Minuten isotherm bei 50 °C, danach Aufheizen mit einer Geschwindigkeit von 5 °C/min auf 240 °C, Gaschromatograph: Hewlett Packard HP 5890) mit internem Standard (Benzonitril): 84,0 % 3-Pentennitril, 4-Pentennitril und 2-Methyl-3-butennitril, bezogen auf eingesetzten Cyanwasserstoff.
Verhältnis 3-Pentennitril : 2-Methyl-3-butennitril = 2,45:1.

### Beispiel 9 (erfindungsgemäß):

### Batch-Hydrocyanierung von 1,3-Butadien unter Einsatz von Ligand Ia

In einem Micro-Rührgefäß werden unter Argon 0,10 g (0,37 mmol) Bis(1,5-cyclooctadien)nickel(0), 0,43 g (1,48 mmol) Ligand Ia und 6,0 g Toluol vorgelegt und 120 Minuten bei Raumtemperatur gerührt. Nach Zugabe von 2,0 g (37 mmol) 1,3-Butadien und 1,0 g (37 mmol) frisch destillierter Blausäure wird das Gefäß mit einem dicht schließenden Septum verschlossen und 5 Stunden auf 80 °C unter Eigendruck erhitzt. Nach dem Erkalten wird der flüssige Reaktionsaustrag analysiert.

**Tabelle 2:**

| Produktverhältnis in GC-Flächen-% | |
|---|---|
| Verbindung | GC-Flächen-% |
| trans-3-Pentennitril | 42,32 |
| 4-Pentennitril | 0,33 |
| cis-3-Pentennitril | 0,63 |
| 2-Methyl-3-butennitril | 18,20 |

Ausbeute (GC, interner Standard Benzonitril): 93 % 3-Pentennitril, 4-Pentennitril und 2-Methyl-3-butennitril, bezogen auf eingesetzten Cyanwasserstoff.
Verhältnis 3-Pentennitril : 2-Methyl-3-butennitril = 2,36:1.
Beispiel 10 (erfindungsgemäß):
Batch-Hydrocyanierung von Styrol mit Ligand Ia

Unter Argon werden in einem Mikro-Rührgefäß 6,0 g Toluol, 0,10 g (0,37 mmol) Bis(1,5-cyclooctadien)nickel(0) und 0,43 g (1,48 mmol) Ligand Ia vorgelegt und bei Raumtemperatur 120 Minuten gerührt. Nach Zugabe von 1,92 g (18,5 mmol) Styrol und 0,5 g (18,5 mmol) frisch destillierter Blausäure wird das Gefäß mit einem dicht schließenden Septum verschlossen und 5 Stunden auf 120 °C temperiert (Eigendruck). Nach Erkalten wird der flüssige Reaktionsaustrag analysiert. Das Gaschromatogramm zeigt 2-Phenylpropionitril (12,53 Fl.-%) und 3-Phenylpropionitril (0,16 Fl.-%) sowie unumgesetztes Styrol (57,9 Fl.-%).

### Beispiel 11 (erfindungsgemäß):

### Semikontinuierliche Hydrocyanierung von 3-Pentennitril mit Ligand Ia

In einer Argonatmosphäre werden 0,87 g (3 mmol) Ligand Ia, 20 ml Toluol und 0,27 g (1 mmol) Bis(1,5-cyclooctadien)-nickel(0) vorgelegt und bei Raumtemperatur 30 Minuten gerührt. Nach Zugabe von 16,2 g (200 mmol) 3-Pentennitril und 0,14 g (1 mmol) ZnCl₂ wird auf 65 °C erwärmt. Über einen geeichten Rotationsverdampfer wird binnen einer Stunde 5,4 g (200 mmol) HCN dest. in einem Argonstrom eingegast. Nach einer Stunde Nachreaktionszeit bei 70 °C wird der flüssige Reaktionsaustrag analysiert. Er enthält 2,87 GC-Flächen-% Adipodintril, 0,72 GC-Flächen-% Methylglutarnitril und 78,5 GC-Flächen-% unumgesetztes 3-Pentennitril.

### Beispiel 12 (erfindungsgemäß):

### Isomerisierung von 2-Methyl-3-butennitril zu 3-Pentennitril mit Ligand Ia

Unter Argonatmosphäre werden 1,2 g (4 mmol) Ligand Ia, 10 ml Toluol und 0,275 g (1 mmol) Bis(1,5-cyclooctadien)nickel(0) vorgelegt und bei Raumtemperatur 30 Minuten gerührt. Nach Zugabe von 8,1 g (100 mmol) 2-Methyl-3-butennitril und 0,55 g (4 mmol) ZnCl₂ wird auf 100 °C erwärmt. Der Reaktionsverlauf wird in regelmäßigen Abständen mittels eines Gaschromatographen untersucht. Das Reaktionsergebnis ist in Tabelle 3 angegeben. Alle dort verzeichneten Produkte und Nebenprodukte wurde zuvor mittels Gaschromatographie, GC-MS, GC-MS-IR sowie mittels NMR zugeordnet. Alle Werte, mit Ausnahme von 3-Pentennitril und 2-Methyl-3-butennitril, sind in GC-Flächen-% angegeben. Bei letzteren wurden die GC-Flächen-% mit Hilfe von Eichmessungen auf Gewichts-% umgerechnet.

**Tabelle 3:**

| Produktverhältnis nach 300 Minuten Reaktionsdauer | | |
|---|---|---|
| | Verbindung | Anteil |
| GC-Flächen-% | trans-2-Methyl-2-butennitril | 2,76 |
| | 2-Methyl-3-butennitril | 2,75 |
| | trans-2-Pentennitril | 0,08 |
| | cis-2-Methyl-2-butennitril | 1,56 |
| | 4-Pentennitril | 0,98 |
| | trans-3-Pentennitril | 29,68 |
| | cis-3-Pentennitril | 1,74 |
| | Benzonitril (Standard) | 52,34 |
| Gew.-% | 2-Methyl-3-butennitril | 3,33 |
| | 3-Pentennitril | 39,19 |

Umsatz: 93,16 %

### Beispiel 13:

### Herstellung eines Nickel(0)-Komplexes aus elementarem Nickel

Die Herstellung eines Phosphonit-Nickel(0)-Komplexes erfolgt ausgehend von Ligand Ia und elementarem Nickelpulver. Unter Argonatmosphäre werden 14,6 g (50 mmol) Ligand Ia, 0,7 g (12,5 mmol) Nickelpulver und 4,7 g 3-Pentennitril vorgelegt und auf 80 °C erwärmt. Nach Zugabe von 2 Tropfen PCl₃ wird 22 Stunden bei 80 °C gerührt. Der rot-braune, viskose Reaktionsaustrag wird nach dem Erkalten über eine Glasfilternutsche abgesaugt. Der durch Elementaranalyse der homogenen Lösung ermittelte Mittelwert beträgt 3,3 Gew.-% (Theorie: 4,78 Gew.-%), entsprechend einer Ausbeute von 69 %, bezogen auf ein gesetztes Nickelpulver.

### Beispiel 14 (erfindungsgemäß):

### Semikontinuierliche Hydrocyanierung von teilhydriertem C₄-Schnitt

**Tabelle 4:**

| Zusammensetzung des C₄-Schnittes | |
|---|---|
| Verbindung | Vol.-% |
| 1,3-Butadien | 38,90 |
| cis-2-Buten | 4,30 |
| trans-2-Buten | 7,05 |
| Isobuten | 22,40 |
| 1-Buten | 19,80 |
| Isobutan | 0,89 |
| n-Butan | 4,50 |
| Propin | 29 ppm |
| Vinylacetylen | 159 ppm |
| 1-Butin | 187 ppm |

In einem Glasautoklaven werden unter Argon bei Raumtemperatur 0,41 g (1,5 mmol) Bis(1,5-cyclooctadien)nickel(0), 2,36 g (6 mmol) Ligand Ib und 6 g Toluol miteinander vermengt. Nach etwa einer Stunde wird eine Mischung aus 20,8 g C₄-Schnitt mit einer Zusammensetzung gemäß Tabelle 4 in 40 g Toluol zugegeben. Der Glasautoklave wird fest verschlossen und die Reaktionsmischung auf 80 °C erhitzt, wobei sich ein Anfangsdruck von 3,2 bar einstellt. Über einen Zeitraum von 120 Minuten wird eine Mischung aus 4,0 g (0,15 mol) frisch destillierter Blausäure in 40 g Toluol kontinuierlich zudosiert. Danach ist der Druck auf 2,3 bar gefallen. Anschließend lässt man zum Vervollständigen der Reaktion noch 100 Minuten bei etwa 80 °C nachreagieren. Zum Nachspülen des Reaktionsaustrages wird Toluol verwendet. Der Verlauf der Reaktion wird über Druck- und Temperaturmessung verfolgt.

Bei einer anschließenden Cyanidbestimmung nach Volhard wird ein Cyanwasserstoffumsatz von 65,3 % ermittelt.

GC-Analytik (Säule: 30 m Stabilwachs, Temperaturprogramm: 5 Minuten isotherm bei 50 °C, danach Aufheizen mit einer Geschwindigkeit von 5 °C/min auf 240 °C, Gaschromatograph Hewlett Packard HP 5890) mit internem Standard (Benzonitril): 64,7 % 3-Pentennitril, 4-Pentennitril und 2-Methyl-3-butennitril, bezogen auf eingesetzten Cyanwasserstoff.

Verhältnis 3-Pentennitril:2-Methyl-3-butennitril = 2,04 : 1.

### Beispiel 15 (erfindungsgemäß):

### Batch-Hydrocyanierung von 1,3-Butadien unter Einsatz von Ligand Ib

In einem Mikro-Rührgefäß werden unter Argon 6,0 g Toluol, 0,10 g (0,37 mmol) Bis(1,5-cyclooctadien)nickel(0) und 0,56 g (1,48 mmol) Ligand Ib vorgelegt und 120 Minuten bei Raumtemperatur gerührt. Nach Zugabe von 2,0 g (37 mmol) 1,3-Butadien und 1,0 g (37 mmol) frisch destillierter Blausäure wird das Gefäß mit einem dicht schließenden Septum verschlossen und 5 Stunden auf 80 °C unter Eigendruck erhitzt. Nach dem Erkalten wird der flüssige Reaktionsaustrag analysiert.

**Tabelle 5:**

| Produktverhältnis in GC-Flächen-% | |
|---|---|
| Verbindung | GC-Flächen-% |
| trans-3-Pentennitril | 40,82 |
| cis-3-Pentennitril | 0,46 |
| 4-Pentennitril | 0,22 |
| cis-2-Methyl-2-butennitril | 0,08 |
| 2-Methyl-3-butennitril | 19,64 |

Ausbeute (GC, interner Standard Benzonitril): 89,2 % 3-Pentennitril, 4-Pentennitril und 2-Methyl-3-Butennitril, bezogen auf eingesetzten Cyanwasserstoff.

Verhältnis 3-Pentennitril:2-Methyl-3-butennitril = 2,1:1.

### Beispiel 16 (erfindungsgemäß):

### Semikontinuierliche Hydrocyanierung von 3-Pentennitril unter Einsatz von Ligand Ib

Unter Argonatmosphäre werden 1,13 g (3 mmol) Ligand Ib, 20 ml Toluol und 0,27 g (1 mmol) Bis(1,5-cyclooctadien)nickel(0) vorgelegt und 30 Minuten bei Raumtemperatur gerührt. Nach Zugabe von 16,2 g (200 mmol) 3-Pentennitril und 0,14 g (1 mmol) ZnCl₂ wird auf 65 °C erwärmt. Über einen geeichten Rotationsverdampfer wird binnen 90 Minuten 5,4 g (200 mmol) frisch destillierte Blausäure in einem Argonstrom eingegast. Nach 1 Stunde Nachreaktionszeit bei 75 °C wird der flüssige Reaktionsaustrag analysiert. Er enthält 2,98 GC-Fl.-% Adipodinitril. 0,67 GC-Fl.-% Methylglutarnitril sowie 76,3 GC-Fl.-% unumgesetztes 3-Pentennitril.

### Beispiel 17 (erfindungsgemäß):

### Isomerisierung von 2-Methyl-3-butennitril zu 3-Pentennitril unter Einsatz von Ligand Ib

Unter Argonatmosphäre werden 1,5 g (4 mmol) Ligand Ib, 10 ml Toluol und 0,275 g (1 mmol) Bis(1,5-cyclooctadien)nickel(0) vorgelegt und 30 Minuten bei Raumtemperatur gerührt. Nach Zugabe von 8,1 g (100 mmol) 2-Methyl-3-butennitril und 0,55 g (4 mmol) ZnCl₂ wird auf 100 °C erwärmt. Die Reaktionsauswertung erfolgt analog zu Beispiel 12. Das Ergebnis ist in der folgenden Tabelle 6 wiedergegeben.

**Tabelle 6:**

| Produktverhältnis nach 300 Minuten Reaktionsdauer | | |
|---|---|---|
| | Verbindung | Anteil |
| GC-Flächen-% | trans-2-Methyl-2-butennitril | 7,69 |
| | 2-Methyl-3-butennitril | 3,87 |
| | trans-2-Pentennitril | 1,96 |
| | cis-2-Methyl-2-butennitril | 15,06 |
| | 4-pentennitril | 0,07 |
| | trans-3-Pentennitril | 16,82 |
| | cis-3-Pentennitril | 0,40 |
| | Benzonitril (Standard) | 51,93 |
| Gew.-% | 2-Methyl-3-butennitril | 4,09 |
| | 3-Pentennitril | 18,20 |

Umsatz: 90,58 %

### Beispiel 18 (erfindungsgemäß):

### Batch-Hydrocyanierung von 1,3-Butadien unter Einsatz von Ligand Ic

In einem Mikro-Rührgefäß werden unter Argon 6,0 g Toluol, 0,10 g (0,37 mmol) Bis(1,5-cyclooctadien)nickel(0) und 0,57 g (1,48 mmol) Ligand Ic vorgelegt und 120 Minuten bei Raumtemperatur gerührt. Nach Zugabe von 2,0 g (37 mmol) 1,3-Butadien und 1,0 g (37 mmol) frisch destillierter Blausäure wird das Gefäß mit einem dicht schließenden Septum verschlossen und 5 Stunden auf 80 °C unter Eigendruck temperiert. Nach dem Erkalten wird der flüssige Reaktionsaustrag mittels Gaschromatographie analysiert. Das Ergebnis ist in der folgenden Tabelle 7 wiedergegeben.

**Tabelle 7:**

| Verbindung und GC-Flächen-% | |
|---|---|
| Verbindung | Vol.-% |
| trans-3-Pentennitril | 18,29 |
| cis-3-Pentennitril | 0,12 |
| cis-2-Methyl-butennitril | 0,03 |
| 2-Methyl-3-butennitril | 8,72 |

Ausbeute (GC, interner Standard Benzonitril): 35,1 % 3-Pentennitril, 4-Pentennitril und 2-Methyl-3-butennitril, bezogen auf eingesetzten Cyanwasserstoff.

Verhältnis 3-Pentennitril:2-Methyl-3-butennitril = 2,11:1.

### Beispiel 19 (erfindungsgemäß):

### Semikontinuierliche Hydrocyanierung von 1,3-Butadien unter Einsatz von Ligand Ic

In einem Glasautoklaven werden unter Argon bei Raumtemperatur 0,41 g (1,5 mmol) Bis(1,5-cyclooctadien)nickel(0), 2,03 g (6 mmol) Ligand Ic und 6 g Toluol miteinander vermengt. Nach etwa 1 Stunde wird eine Mischung aus 8,1 g 1,3-Butadien in 40 g Toluol zugegeben. Der Glasautoklave wird fest verschlossen und die Reaktionsmischung auf 110 °C temperiert, wobei sich ein Anfangsdruck von 2,0 bar einstellt. Über einen Zeitraum von 120 Minuten wird eine Mischung aus 4,0 g (0,15 mmol) frisch destillierter Blausäure in 40 g Toluol kontinuierlich zudosiert. Danach ist der Druck auf 1,5 bar gefallen. Anschließend lässt man zur Vervollständigung der Reaktion noch 310 Minuten bei etwa 110 °C nachreagieren. Zum Nachspülen des Reaktionsaustrags wird Toluol verwendet. Der Verlauf der Reaktion wird über Druck- und Temperaturmessung verfolgt. Bei einer anschließenden Cyanidbestimmung nach Volhard wird ein Cyanwasserstoffumsatz von 74,3 % ermittelt.

GC-Analytik (Säule: 30 m Stabilwachs, Temperaturprogramm: 5 Minuten isotherm bei 50 °C, anschließendes Aufheizen auf 240 °C mit einer Geschwindigkeit von 5 °C/min, Gaschromatograph: Hewlett Packard HP 5890) mit internem Standard (Benzonitril): 72,5 % 3-Pentennitril, 4-Pentennitril und 2-Methyl-3-butennitril, bezogen auf eingesetzten Cyanwasserstoff.

Verhältnis 3-Pentennitril:2-Methyl-3-butennitril = 1,89:1.

### Beispiel 20 (erfindungsgemäß):

### Semikontinuierliche Hydrocyanierung von 3-Pentennitril unter Einsatz von Ligand Ic

Unter Argonatmosphäre werden 1,15 g (3 mmol) Ligand Ic, 20 ml Toluol und 0,27 g (1 mmol) Bis(1,5-cyclooctadien)nickel(0) vorgelegt und 30 Minuten bei Raumtemperatur gerührt. Nach Zugabe von 24,3 g (300 mmol) 3-Pentennitril und 0,14 g (1 mmol) ZnCl₂ wird auf 60 °C erwärmt. Über einen geeichten Rotationsverdampfer wird binnen 2 Stunden 5,4 g (200 mmol) destillierte Blausäure in einem Argonstrom eingegast. Nach einer Stunde Nach-reaktionszeit bei 70 °C wird der flüssige Reaktionsaustrag analysiert. Er enthält 1,66 GC-Fl.-% Adipodinitril, 0,34 GC-Fl.-% Methylglutarnitril sowie 82,3 GC-Fl.-% unumgesetztes 3-Pentennitril.

### Beispiel 21 (erfindungsgemäß):

### Batch-Hydrocyanierung von 1,3-Butadien unter Einsatz von Ligand Id

In einem Mikro-Rührgefäß werden unter Argon 6,0 g Toluol, 0,10 g (0,37 mmol) Bis(1,5-cyclooctadien)nickel(0) und 0,46 g (1,48 mmol) Ligand Id vorgelegt und 120 Minuten bei Raumtemperatur gerührt. Nach Zugabe von 2,0 g (37 mmol) 1,3-Butadien und 1,0 g (37 mmol) frisch destillierter Blausäure wird das Gefäß mit einem dicht schließenden Septum verschlossen und 5 Stunden auf 80 °C unter Eigendruck temperiert. Nach dem Erkalten wird der flüssige Reaktionsaustrag analysiert.

Ausbeute (GC, interner Standard Benzonitril): 8,9 % 3-Pentennitril, 4-Pentennitril und 2-Methyl-3-butennitril, bezogen auf eingesetzten Cyanwasserstoff.

Verhältnis 3-Pentennitril:2-Methyl-3-butennitril = 2,1:1.

### Beispiel 22 (erfindungsgemäß):

### Semikontinuierliche Hydrocyanierung von 3-Pentennitril unter Einsatz von Ligand Id

Unter Argonatmosphäre werden 0,93 g (3 mmol) Ligand Id, 20 ml Toluol und 0,27 g (1 mmol) Bis(1,5-cyclooctadien)nickel(0) vorgelegt und 30 Minuten bei Raumtemperatur gerührt. Nach Zugabe von 16,2 g (200 mmol) 3-Pentennitril und 0,14 g (1 mmol) ZnCl₂ wird auf 60 °C erwärmt. Über einen geeichten Rotationsverdampfer wird binnen 2 Stunden 5,4 g (200 mmol) destillierte Blausäure in einem Argonstrom eingegast. Nach 1 Stunde Nachreaktionszeit bei 70 °C wird der flüssige Reaktionsaustrag analysiert. Er enthält 1,01 GC-Fl.-% Adipodinitril, 0,25 GC-Fl.-% Methylglutarnitril sowie 84,99 GC-Fl.-% unumgesetztes 3-Pentennitril.

### Beispiel 23 (erfindungsgemäß):

### Batch-Hydrocyanierung von 1,3-Butadien unter Einsatz von Ligand Ie

In einem Mikro-Rührgefäß werden unter Argon 6,0 g Toluol, 0,10 g (0,37 mmol) Bis(1,5-cyclooctadien)nickel(0) und 0,62 g (1,8 mmol) Ligand Ie vorgelegt und 120 Minuten bei Raumtemperatur gerührt. Nach Zugabe von 2,0 g (37 mmol) 1,3-Butadien und 1,0 g (37 mmol) frisch destillierter Blausäure wird das Gefäß mit einem dicht schließenden Septum verschlossen und 5 Stunden auf 80 °C unter Eigendruck temperiert. Nach dem Erkalten wird der flüssige Reaktionsaustrag analysiert.

Ausbeute (GC, interner Standard Benzonitril): 9,7 % 3-Pentennitril, 4-Pentennitril und 2-Methyl-3-butennitril, bezogen auf eingesetzten Cyanwasserstoff.

Verhältnis 3-Pentennitril:2-Methyl-3-butennitril = 1,94:1.

### Beispiel 24 (erfindungsgemäß):

### Semikontinuierliche Hydrocyanierung von 3-Pentennitril unter Einsatz von Ligand Ie

Unter Argonatmosphäre werden 1,26 g (3 mmol) Ligand Ie, 20 ml Toluol und 0,27 g (1 mmol) Bis(1,5-cyclooctadien)nickel(0) vorgelegt und 30 Minuten bei Raumtemperatur gerührt. Nach Zugabe von 16,2 g (200 mmol) 3-Pentennitril und 0,14 g (1 mmol) ZnCl₂ wird auf 60 °C erwärmt. Über einen geeichten Rotationsverdampfer wird binnen 2 Stunden 5,4 g (200 mmol) destillierte Blausäure in einem Argonstrom eingegast. Nach 1 Stunde Reaktionszeit bei 70 °C wird der flüssige Reaktionsaustrag analysiert. Er enthält 1,06 GC-Fl.-% Adipodinitril, 0,24 GC-Fl.-% Methylglutarnitril sowie 83,7 GC-Fl.-% unumgesetztes 3-Pentennitril.

## Patentansprüche

1. Katalysator, umfassend einen Nickel(O)komplex, mit einem einzähnigen Phosphonitliganden der allgemeinen Formel I 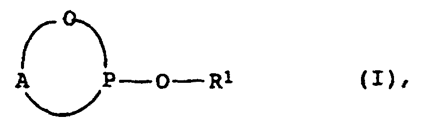 worin
A zusammen mit dem Teil der Phosphonitgruppe, an den es gebunden ist, für einen 5- bis 8-gliedrigen Heterocyclus steht, der gegebenenfalls zusätzlich ein-, zwei- oder dreifach mit Cycloalkyl, Aryl und/oder Hetaryl anelliert sein kann, wobei die anellierten Gruppen je einen, zwei oder drei Substituenten, ausgewählt unter Alkyl, Alkoxy, Halogen, Nitro, Cyano oder Carboxyl tragen können,
R¹ für Alkyl, Aryl oder Hetaryl steht, welche einen, zwei oder drei der folgenden Substituenten: Alkyl, Cycloalkyl, Aryl, Alkoxy, Cycloalkyloxy, Acyl, Aryloxy, Halogen, Trifluormethyl, Nitro, Cyano, Carboxyl oder NE¹E² tragen können, wobei E¹ und E² gleich oder verschieden sein können und für Alkyl, Cycloalkyl oder Aryl stehen,
oder Salze und Mischungen davon.

2. Katalysator nach Anspruch 1, wobei A zusammen mit dem Teil der Phosphonitgruppe, an den es gebunden ist, für einen 5-oder 6-gliedrigen Heterocyclus steht, der gegebenenfalls einoder zweifach mit Aryl und/oder Hetaryl anelliert sein kann, wobei die anellierten Gruppen einen, zwei oder drei der zuvor angegebenen Substituenten tragen können.

3. Katalysator nach einem der vorhergehenden Ansprüche, wobei A für einen Rest der Formel IV.1 oder IV.2 oder IV.3 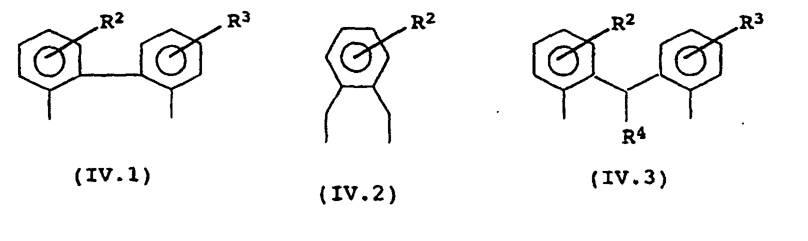 steht,
worin
R² und R³ unabhängig voneinander für Wasserstoff, Alkyl, Alkoxy, Halogen, Trifluormethyl, Nitro oder Cyano stehen,
R⁴ für Wasserstoff, Alkyl, vorzugsweise Methyl, oder Aryl, vorzugsweise Phenyl, steht, welches gegebenenfalls durch Alkyl, Alkoxy, Halogen, Trifluormethyl, Nitro oder Cyano substituiert sein kann.

4. Katalysator nach einem der vorhergehenden Ansprüche, wobei
R¹ für Phenyl oder Naphthyl steht, welches einen, zwei oder drei der folgenden Substituenten: Alkyl, Alkoxy, Halogen, Nitro, Cyano, Carboxyl oder NE¹E² tragen kann, wobei E¹ und E² die zuvor angegebenen Bedeutungen besitzen,

5. Katalysator nach einem der vorhergehenden Ansprüche, wobei der Phosphonitligand der Formel I ausgewählt ist unter Liganden der Formeln Ia bis Ie 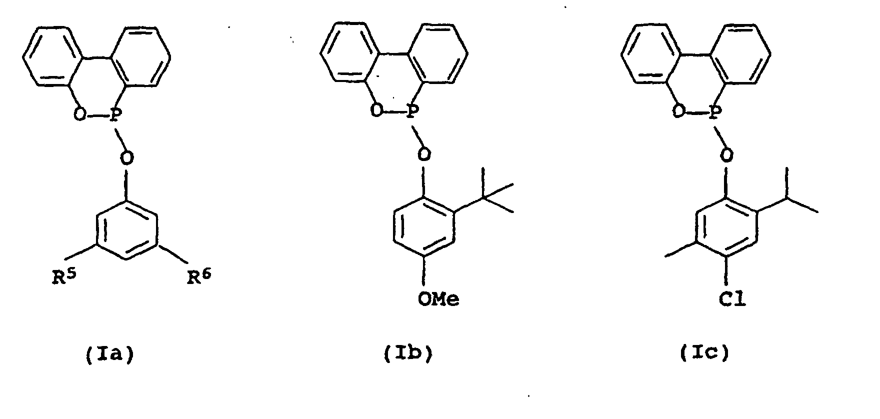 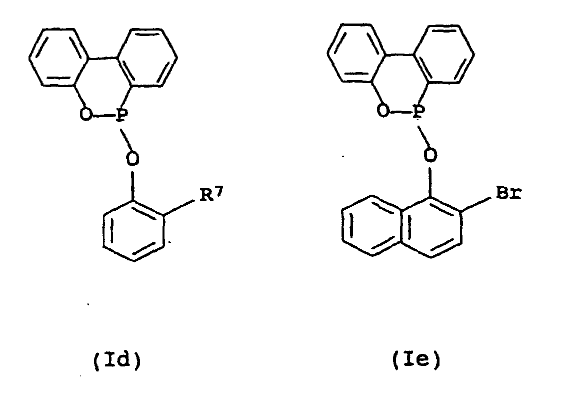 worin
R⁵ und R⁶ unabhängig voneinander für Wasserstoff oder Trifluormethyl stehen,
R⁷ für Fluor oder Trifluormethyl steht.

6. Verfahren zur Herstellung der Katalysatoren nach einem der vorhergehenden Ansprüche, **dadurch gekennzeichnet, dass** man mindestens einen Phosphonitliganden der Formel I mit Nickel oder einer Nickelverbindung in Gegenwart eines Reduktionsmittels oder einem Nickel(0)komplex in einem inerten Lösungsmittel zur Reaktion bringt.

7. Verfahren zur Herstellung von Gemischen monoolefinischer C₅-Mononitrile mit nichtkonjugierter C=C- und C≡N-Bindung durch katalytische Hydrocyanierung von Butadien oder eines 1,3-Butadien-haltigen Kohlenwasserstoffgemisches, **dadurch gekennzeichnet, dass** die Hydrocyanierung in Gegenwart eines Katalysators nach einem der Ansprüche 1 bis 5 erfolgt.

8. Verfahren nach Anspruch 7, **dadurch gekennzeichnet, dass** man ein Kohlenwasserstoffgemisch mit einem 1,3-Butadiengehalt von mindestens 10 Vol.-%, bevorzugt mindestens 25 Vol.-%, insbesondere mindestens 40 Vol.-%, einsetzt.

9. Verfahren nach einem der Ansprüche 7 oder 8, **dadurch gekennzeichnet, dass** man als 1,3-Butadien-haltiges Kohlenwasserstoffgemisch einen C₄-Schnitt aus der Erdölverarbeitung einsetzt.

10. Verfahren nach einem der Ansprüche 7 bis 9, **dadurch gekennzeichnet, daß** man ein Produktgemisch erhält, welches isomere Pentennitrile und Methylbutennitrile, wie 3-Pentennitril, 4-Pentennitril, 2-Methyl-2-butennitril umfasst.

11. Verfahren nach Anspruch 10, **dadurch gekennzeichnet, dass** das Mengenverhältnis von 3-Pentennitril zu 2-Methyl-3-butennitril mindestens 1,9:1, bevorzugt mindestens 2,1:1 beträgt.

12. Verfahren nach einem der Ansprüche 7 bis 11, **dadurch gekennzeichnet, dass** der Katalysator zusätzlich zur Hydrocyanierung auch zur Stellungs- und Doppelbindungsisomerisierung des Kohlenwasserstoffgemisches und/oder der monoolefinischen C₅-Mononitrile eingesetzt wird.

13. Verfahren zur Herstellung von Adipodinitril, **dadurch gekennzeichnet, daß** man ein gemäß den Ansprüchen 7 bis 12 hergestelltes Gemisch von C₅-Mononitrilen, gegebenenfalls nach weiterer Aufarbeitung und/oder Isomerisierung in Gegenwart eines Katalysators nach einem der Ansprüche 1 bis 5 katalytisch hydrocyaniert.

14. Verwendung von Katalysatoren, umfassend einen Liganden der Formel I zur Hydrocyanierung und/oder Stellungs- und Doppelbindungsisomerisierung von Olefinen.

## Claims

1. A catalyst comprising a nickel(0) complex having a monodentate phosphonite ligand of the formula I 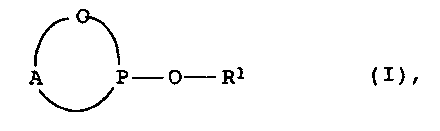 where
A is, together with the part of the phosphonite group to which it is bonded, a 5- to 8-membered heterocycle which may additionally be fused once, twice or three times to cycloalkyl, aryl and/or hetaryl, where the fused groups may in each case have one, two or three substituents selected from alkyl, alkoxy, halogen, nitro, cyano or carboxyl,
R¹ is alkyl, aryl or hetaryl, each of which may have one, two or three of the following substituents: alkyl, cycloalkyl, aryl, alkoxy, cycloalkyloxy, acyl, aryloxy, halogen, trifluoromethyl, nitro, cyano, carboxyl or NE¹E², where E¹ and E² may be identical or different and are alkyl, cycloalkyl or aryl,
or salts and mixtures thereof.

2. A catalyst as claimed in claim 1, where A is, together with the part of the phosphonite group to which it is bonded, a 5- or 6-membered heterocycle which may be fused once or twice to aryl and/or hetaryl, where the fused groups may have one, two or three of the abovementioned substituents.

3. A catalyst as claimed in either of the preceding claims, where A is a radical of the formula IV.1 or IV.2 or IV.3 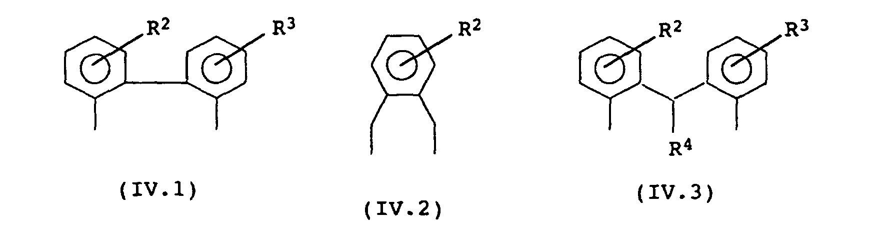 where
R² and R³ are, independently of one another, hydrogen, alkyl, alkoxy, halogen, trifluoromethyl, nitro or cyano,
R⁴ is hydrogen, alkyl, preferably methyl, or aryl, preferably phenyl, which may be substituted by alkyl, alkoxy, halogen, trifluoromethyl, nitro or cyano.

4. A catalyst as claimed in any of the preceding claims, where
R¹ is phenyl or naphthyl, which may have one, two or three of the following substituents: alkyl, alkoxy, halogen, nitro, cyano, carboxyl or NE¹E², where E¹ and E² have the abovementioned meanings

5. A catalyst as claimed in any of the preceding claims, where the phosphonite ligand of the formula I is selected from ligands of the formulae Ia to Ie 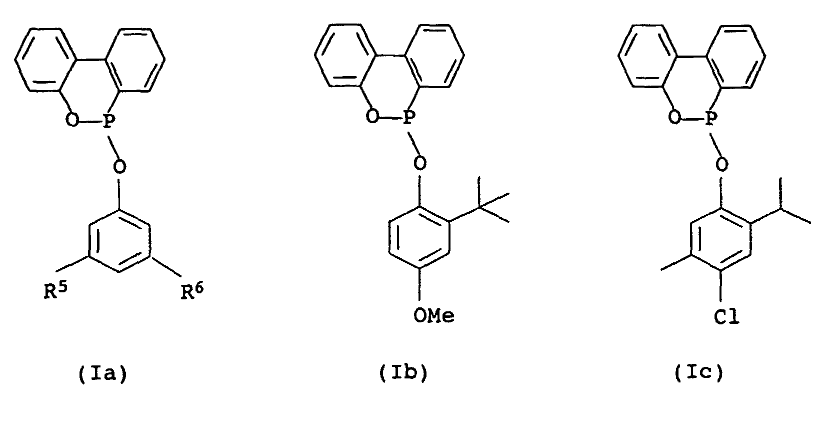 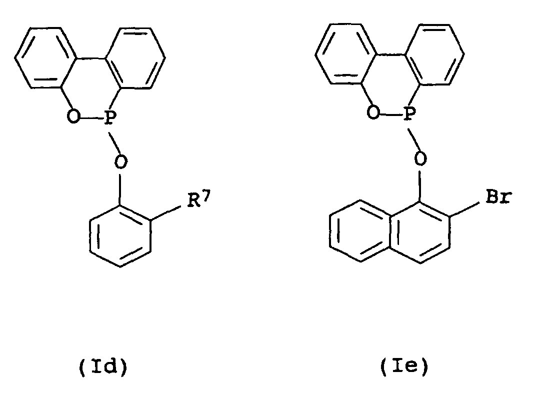 where
R⁵ and R⁶, independently of one another, are hydrogen or trifluoromethyl,
R⁷ is fluorine or trifluoromethyl.

6. A process for preparing a catalyst as claimed in any of the preceding claims, which comprises reacting at least one phosphonite ligand of the formula I with nickel or a nickel compound in the presence of a reducing agent or with a nickel(0) complex in an inert solvent.

7. A process for preparing mixtures of monoolefinic C₅ mononitriles with nonconjugated C=C and C≡N bonds by catalytic hydrocyanation of butadiene or of a 1,3-butadiene-containing hydrocarbon mixture, which comprises carrying out the hydrocyanation in the presence of a catalyst as claimed in any of claims 1 to 5.

8. A process as claimed in claim 7, wherein a hydrocarbon mixture with a 1,3-butadiene content of at least 10% by volume, preferably at least 25% by volume, in particular at least 40% by volume, is employed.

9. A process as claimed in either of claims 7 or 8, wherein a C₄ cut from petroleum processing is employed as 1,3-butadiene-containing hydrocarbon mixture.

10. A process as claimed in any of claims 7 to 9, wherein the resulting product mixture comprises isomeric pentenonitriles and methylbutenonitriles, such as 3-pentenonitrile, 4-pentenonitrile and 2-methyl-2-butenonitrile.

11. A process as claimed in claim 10, wherein the ratio of the amounts of 3-pentenonitrile and 2-methyl-3-butenonitrile is at least 1.9:1, preferably at least 2.1:1.

12. A process as claimed in any of claims 7 to 11, wherein, in addition to the hydrocyanation, the catalyst is also employed for the positional and double-bond isomerization of the hydrocarbon mixture and/or of the monoolefinic C₅ mononitriles.

13. A process for preparing adiponitrile, which comprises catalytic hydrocyanation of a mixture, prepared as claimed in claims 7 to 12, of C₅ mononitriles, where appropriate after further workup and/or isomerization in the presence of a catalyst as claimed in any of claims 1 to 5.

14. The use of catalysts comprising a ligand of the formula I for the hydrocyanation and/or positional and double-bond isomerization of olefins.

## Revendications

1. Catalyseur, comprenant un complexe de nickel(0) à base d'un ligand phosphonite monodenté de la formule générale I 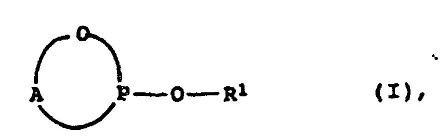 dans laquelle
A représente, avec la partie du groupe phosphonite à laquelle il est lié, un hétérocycle à 5 à 8 chaînons, qui peut éventuellement être annélé une, deux ou trois fois avec un radical cycloalkyle, un aryle et/ou un hétaryle, les groupes annélés pouvant chacun porter un, deux ou trois substituants, choisis parmi des radicaux alkyle, alcoxy, halogène, nitro, cyano ou carboxyle,
R¹ représente des radicaux alkyle, aryle ou hétaryle, qui peuvent porter un, deux ou trois des substituants suivants: alkyle, cycloalkyle, aryle, alcoxy, cycloalcoxy, acyle, aryloxy, halogène, trifluorométhyle, nitro, cyano, carboxyle ou NE¹E², où E¹ et E² peuvent être identiques ou différents et représenter un alkyle, un cycloalkyle ou un aryle,
ou leurs sels ou leurs mélanges.

2. Catalyseur selon la revendication 1, dans lequel A représente, avec la partie du groupe phosphonite à laquelle il est lié, un hétérocycle à 5 ou 6 chaînons, qui peut éventuellement être annélé une ou deux fois avec un aryle et/ou un hétaryle, les groupes annélés pouvant porter un, deux ou trois des substituants mentionnés plus haut.

3. Catalyseur selon l'une quelconque des revendications qui précèdent, dans lequel A représente un reste de formule IV.1 ou IV.2 ou IV.3 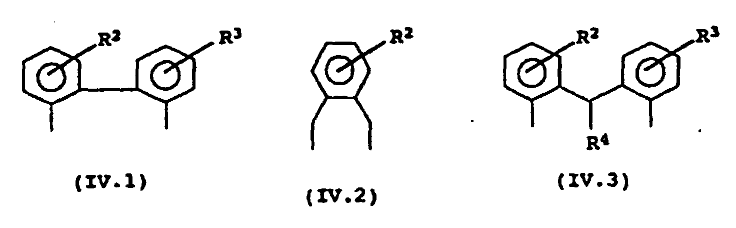 dans lesquelles
R² et R³ représentent indépendamment l'un de l'autre de l'hydrogène ou des radicaux alkyle, alcoxy, halogène, trifluorométhyle, nitro ou cyano,
R⁴ représente de l'hydrogène ou des radicaux alkyle, de préférence méthyle, ou aryle, de préférence phényle, qui peut éventuellement être substitué par des radicaux alkyle, alcoxy, halogène, trifluorométhyle, nitro ou cyano.

4. Catalyseur selon l'une quelconque des revendications qui précèdent, dans lequel
R¹ représente un radical phényle ou naphtyle, qui peut porter un, deux ou trois des substituants suivants: alkyle, alcoxy, halogène, nitro, cyano, carboxyle ou NE¹E², où E¹ et E² ont les significations indiquées plus haut.

5. Catalyseur selon l'une quelconque des revendications qui précèdent, dans lequel le ligand phosphonite de la formule I est choisi parmi des ligands des formules la à le 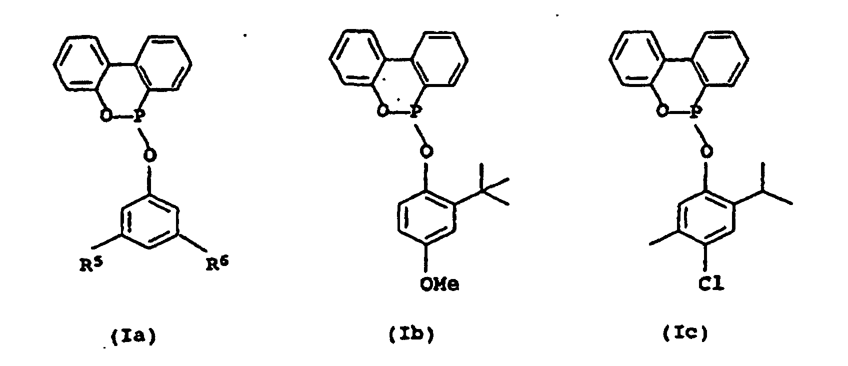 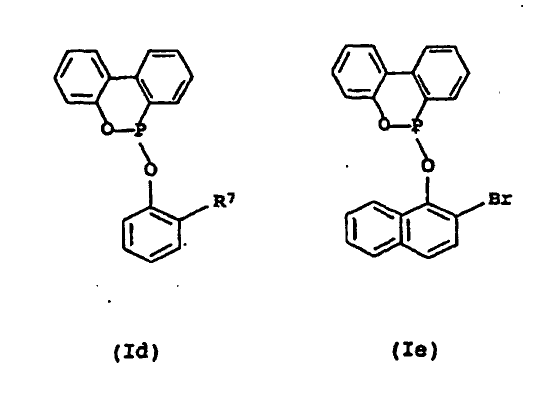 dans lesquelles
R⁵ et R⁶ représentent indépendamment l'un de l'autre de l'hydrogène ou un radical trifluorométhyle,
R⁷ représente du fluor ou un radical trifluorométhyle.

6. Procédé de préparation des catalyseurs selon l'une quelconque des revendications qui précèdent, **caractérisé en ce que** l'on fait réagir au moins un ligand phosphonite de la formule 1 avec du nickel ou un composé de nickel en présence d'un réducteur ou d'un complexe de nickel(0), dans un solvant inerte.

7. Procédé de préparation de mélanges de mononitriles en C₅ monooléfiniques comportant des liaisons C=C et C≡N non conjuguées, par hydrocyanation catalytique de butadiène ou d'un mélange d'hydrocarbures contenant du 1,3-butadiène, **caractérisé en ce que** l'hydrocyanation est entreprise en présence d'un catalyseur selon l'une quelconque des revendications 1 à 5.

8. Procédé selon la revendication 7, **caractérisé en ce que** l'on met en oeuvre un mélange d'hydrocarbures d'une teneur en 1,3-butadiène d'au moins 10% en volume, de préférence d'au moins 25% en volume, en particulier d'au moins 40% en volume.

9. Procédé selon l'une quelconque des revendications 7 ou 8, **caractérisé en ce que** l'on met en oeuvre en tant que mélange d'hydrocarbures contenant du 1,3-butadiène une coupe en C₄ provenant du traitement de pétrole.

10. Procédé selon l'une quelconque des revendications 7 à 9, **caractérisé en ce que** l'on obtient un mélange de produits comprenant des pentène-nitriles et des méthylbutène-nitriles isomères, comme du 3-pentène-nitrile, du 4-pentène-nitrile et du 2-méthyl-2-butène-nitrile.

11. Procédé selon la revendication 10, **caractérisé en ce que** la proportion quantitative du 3-pentène-nitrile par rapport au 2-méthyl-3-butène-nitrile est d'au moins 1,9 : 1, de préférence d'au moins 2,1 : 1.

12. Procédé selon l'une quelconque des revendications 7 à 11, **caractérisé en ce que** le catalyseur est, outre l'hydrocyanation, également mis en oeuvre pour l'isomérisation de position et de double liaison du mélange d'hydrocarbures et/ou des mononitriles monooléfiniques en C₅.

13. Procédé de préparation d'adiponitrite, **caractérisé en ce que** l'on opère l'hydrocyanation catalytique d'un mélange de mononitriles en C₅ préparé selon les revendications 7 à 12, éventuellement après un autre traitement et/ou une isomérisation, en présence d'un catalyseur selon l'une quelconque des revendications 1 à 5.

14. Utilisation de catalyseurs comprenant un ligand de formule I pour l'hydrocyanation et/ou l'isomérisation de position et/ou de double liaison d'oléfines.
